# EUROPEAN PATENT APPLICATION

(11) **EP 2 520 292 A1**
(43) Date of publication of application: **07.11.2012**
(21) Application number: 11003737.1
(22) Date of filing: 06.05.2011
(51) Int. Cl.: A61K 31/35, A61P 17/00, A61P 25/00, A61P 29/00, A61P 35/00

(54) **Use of spirangiens for the treatment or prevention of IL-8 or IL-6 mediated disorders**

(71) Applicant: Helmholtz-Zentrum für Infektionsforschung GmbH, 38124 Braunschweig (DE)
(72) Inventor: Reboll, Marc, 30519 Hannover (DE); Nourbakhsh, Mahtab, D-38124 Braunschweig (DE); Niggemann, Jutta, 38102 Braunschweig (DE); Gerth, Klaus, 38124 Braunschweig (DE); Steinmetz, Heinrich, 31139 Hildesheim (DE); Frank, Ronald, 38527 Meine (DE)
(74) Representative: Forstmeyer, Dietmar

(57) **Abstract**

The invention relates to spirangien for use in the treatment or prevention of an Interleukin 6 (IL-6) or Interleukin 8 (IL-8) mediated disorder. The invention also provides a pharmaceutical formulation for use in the treatment or prevention of an IL-6 or IL-8 mediated disorder and a method of preventing or treating an IL-6 or IL-8 mediated disorder.

## Description

### Field of the Invention

The invention relates to spirangien for use in the treatment or prevention of an Interleukin 6 (IL-6) or Interleukin 8 (IL-8) mediated disorder. The invention also provides a pharmaceutical formulation for use in the treatment or prevention of an IL-6 or IL-8 mediated disorder and a method of preventing or treating an IL-6 or IL-8 mediated disorder.

### Background of the Invention

### Spirangiens

In the past three decades myxobacteria were revealed as versatile producers of biologically active secondary metabolites with unique structures and numerous different mechanisms of action (Wenzel, S. C.; Müller, R. Curr.Opin.Drug Discovery & Devel. 2009, 12, 220-230; Weissman, K. J.; Müller, R. Nat.Prod.Rep. 2010, 27, 1276-95). Many of these secondary metabolites from myxobacteria show antimicrobial or cytotoxic activities. The family of spirangiens was first described by Höfle and co-workers (DE 4211056; Niggemann, J. et al. Eur.J.Org.Chem. 2005, 23, 5013-5018). The total synthesis of spirangiens and derivatives thereof has also been reported (Paterson, I. et al. Chem. Commun. 2008, 47, 6408-6410; Niggemann, J, et al loc. cit). Structurally, the spirangiens possess more than 10 stereocentres, and include a highly functionalized spiroketal core structure.

### Cytokines

Cytokines are soluble proteins, peptides or glycoproteins that are released by different types of cells. Cytokines exert differential effects by autocrine and/or paracrine regulation mechanisms. In case of an autocrine regulation mechanism the endogenous produced cytokine affects the same type of expressing cells. In case of a paracrine regulation mechanism cytokines exert a differential effect on none expressing cells. Cytokines act by binding to their specific receptors and causes a change in function or in development (differentiation) of the target cell. In both cases, i.e. autocrine and paracrine regulation mechanisms the expression of the membrane receptor can be altered. Interleukins are a group of cytokines which are produced by a wide variety of body cells. The majority of interleukins are synthesized by helper CD4+ T lymphocytes, monocytes/macrophages and by endothelial cells. Following synthesis, interleukins are measurable in the producing cells, even after release and secretion in the supernatant of cells, in blood or in body fluids.

Interleukin-6 (IL-6) is a pleiotropic cytokine with biological effects on many cell types. IL-6 is produced by monocytes/macrophages, fibroblasts, endothelial cells, keratinocytes, B and T cells, mast cells, polymorphonuclear neutrophils, some nerve cells, muscle cells, adipocytes and certain tumour cells. IL-6 exits in three molecular weights, i.e. 21-30 kDa, 150-200 or 450 kDa. The biological activity of IL-6 depends on binding to its specific receptors. IL-6 is often regarded as being downstream of TNF or IL-1 in inflammatory cytokine cascades and therefore represents a common pathway factor in a wide range of inflammatory processes. IL-6 is relevant to many disorders such as diabetes, atherosclerosis, depression, Alzheimer's Disease, systemic lupus erythematosus, prostate cancer, and rheumatoid arthritis. Accordingly, blockade of IL-6 synthesis and signaling offers the potential to ameliorate IL-6 mediated disorders.

Interleukin 8 (IL-8) is a cytokine that displays chemotactic activity for specific types of leukocytes. IL-8 is produced by monocytes/macrophages, neutrophils, lymphocytes, dermal fibroblasts, keratinocytes, vascular endothelial cells, melanocytes, and hepatocytes. IL-8 is a member of the CXC chemokine family polypeptides and exists in two predominant forms consisting of 72 amino acids and 77 amino acids, respectively. IL-8 participates in the migration of neutrophils towards inflammatory sites. Upon binding to its high-affinity receptors (CXCR1 and CXCR2) which are present on the surface of neutrophils, IL-8 activates neutrophils by accelerating degranulation and elevating the concentration of free calcium ions in the cytoplasm and also induces neutrophil migration to thereby destroy the infiltrated tissue. IL-8 is relevant to many disorders such as inflammatory or hyperproliferative skin disorders, immune, autoimmune, inflammatory or infectious diseases. IL-8 contributes to human melanoma progression by functioning as a mitogenic and angiogenic factor. Accordingly, blockade of IL-8 synthesis and signaling offers the potential to ameliorate IL-8 mediated disorders and to control angiogenesis, growth, and metastasis of melanoma.

Potent and selective anti-IL-6 and -IL-8 agents have been developed and reported for the treatment of the above pathophysiological situations, e.g. the anti-IL-6 agent tocilizumab, which has been approved for rheumatoid arthritis, and ALD518. Other agents are, for example, described in WO 2002/30353; WO 2003/29242; WO 2003/10163; and WO 2001/58890. Moreover, antibodies specific to IL-6 or IL-8 have been developed and reported as therapeutically useful and important agents for treating disorders mediated by IL-6 and IL-8 activity, respectively. Such antibodies are, for example, described in EP 1590364; EP 1874820; US 6,300,129; US 5,888,510; US 6,723,319; and US 2001/0001663.

Thus, although a number of agents exist or are currently under development for the treatment of IL-6 or IL-8 mediated disorders, there is still a need for additional agents that can be used for the treatment of IL-6 or IL-8 mediated disorders, since many of the known agents show severe side effects on the patient, low drug interaction, or limited bioavailability especially with regard to oral administration, metabolic stability, and solubility. For instance, antibodies specific to IL-6 or IL-8 cannot be orally administered and may elicit an adverse immune response in a patient upon application. Further, production of antibodies specific to IL-6 or IL-8 is complicated and laborious, and thus costly. As a result, a need remains to provide easily applicable methods and agents that may be used to effectively treat IL-6 or IL-8.

It is, therefore, an aim of the present invention to provide more effective and less life threatening agents for use in the treatment or prevention of an IL-8 or IL-6 mediated disorder.

### Summary and Description of the Invention

The present invention was made in view of the prior art and the needs described above, and, therefore, the object of the present invention is to provide novel alternative agents for use in the treatment or prevention of an IL-8 or IL-6 mediated disorder.

Other objects of the present invention are to provide a pharmaceutical composition for use in the treatment or prevention of an IL-6 or IL-8 mediated disorder, comprising at least one spirangien, to provide a combinatorial composition, comprising at least one spirangien and at least one further active pharmaceutical ingredient, and to provide methods for treating patients suffering from an IL-6 or IL-8 mediated disorder.

These objects are solved by the subject matter of the attached claims.

These and other aspects of the present invention will become apparent upon reference to the following detailed description and definitions.

There is accumulating evidence that neutralizing the biological activity of cytokines which significantly contribute to a number of disorders, which are characterized by aberrant overproduction of these cytokines, does reduce the clinical disease activity in patients suffering from such disorders. For example, the study of Skov et al. J.Immunol. 2008, 181, 669-679 provides in vivo proof of concept for the application of an anti-human IL-8 antibody for the treatment of inflammatory diseases.

The inventors showed that spirangiens inhibit the expression of both IL-6 and IL-8 mRNAs and proteins.

The inventors showed that the inhibition of IL-6 and IL-8 expression by spirangiens is dose dependent.

The inventors also showed that spirangiens exhibit no acute toxicity at effective concentrations.

These results for the first time allow a therapeutic, preventive and/or curative role to be conceived for spirangiens in the treatment or prevention of an IL-6 or IL-8 mediated disorder.

Accordingly, the present invention is directed to a spirangien for use in the treatment or prevention of an IL-6 or IL-8 mediated disorder, wherein the spirangien is a compound according to general formula (I) or (II), or a salt thereof: 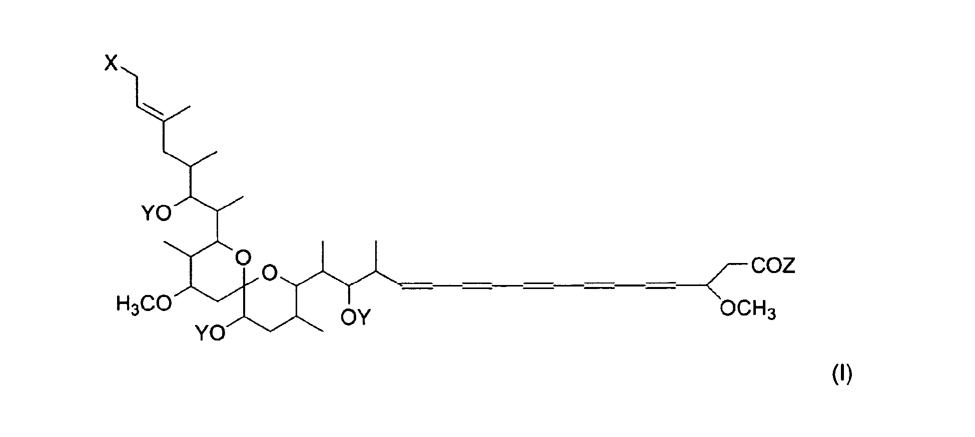 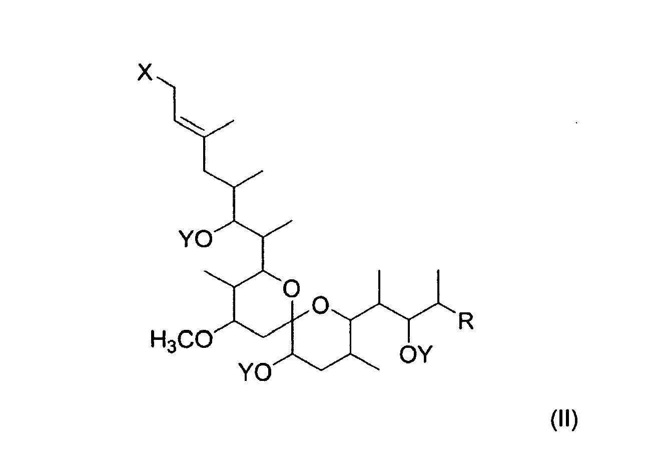 wherein
R is a C₂, C₄ or C₆ alkenyl group;
X is a hydrogen atom or a methyl group;
Y is a hydrogen atom, a C₁-C₅ alkyl, or a C₁-C₅ acyl group;
Z is a OH group; a C₁-C₅ alkoxy group; or a NH₂ group, optionally substituted with one or two C₁-C₅ alkyl groups.

By "treatment or prevention" is meant a reduction or complete inhibiton of the biological activity of IL-6 and/or IL-8 in a subject to thereby cure or prevent the symptoms associated with disorders that are mediated by IL-6 and/or IL-8. The reduction or complete inhibition of the biological activity of IL-6 and/or IL-8 is caused by a reduced expression of IL-6 and/or IL-8 at the protein and/or mRNA level.

The phrase "IL-6 or IL-8 mediated disorder" means disorders which lead to or are mediated by an aberrant synthesis of IL-6 or IL-8.

The term "alkyl" refers to a saturated, straight-chain or branched hydrocarbon group, containing the indicated number of carbon atoms. Examples of alkyl groups are a methyl, ethyl, propyl, iso-propyl, n-butyl, iso-butyl, sec-butyl, tert-butyl, or n-pentyl group.

The term "alkenyl" refers to an at least partially unsaturated, straight-chain or branched hydrocarbon group, with one, two, three or more carbon-carbon double bond(s), containing the indicated number of carbon atoms. Examples of alkenyl groups are ethenyl, butenyl, butadienyl, hexenyl, hexadienyl, and hexatrienyl.

The term "acyl group" refers to a hydrocarbon group, e. g. alkyl or alkenyl group, preferably an alkyl group, that is attached to a CO group via a single carbon-carbon bond. Examples of acyl groups are formyl, acetyl, propionyl, butyryl, iso-butyryl or valeryl.

The term "alkoxy group" refers to a hydrocarbon group, e. g. alkyl or alkenyl group, preferably an alkyl group, singular bonded to oxygen, thus -O-alkyl or -O-alkenyl. Examples of C₁-C₅ alkoxy groups are methoxy, ethoxy, n-propyloxy-, iso-propyloxy-, n-butoxy, iso-butoxy, tert-butoxy, or pentoxy.

As used herein a wording defining the limits of a range such as, e. g., "C₁-C₅" means any integer from 1 to 5, i. e. C₁, C₂, C₃, C₄, and C₅. In other words, any range defined by two integers explicitly mentioned is meant to comprise and disclose any integer defining said limits and any integer comprised in said range.

Preferably, the spirangien for use in the treatment or prevention of an IL-6 or IL-8 mediated disorder is a spirangien according to general formula (I), wherein X, Y and Z each represent hydrogen atom (spirangien A); or a spirangien according to general formula (II), wherein R is butadienyl, and X, Y and Z each represent hydrogen atom (spirangien M522).

Preferably, the IL-6 or IL-8 mediated disorder that can be treated or prevented using spirangien according to general formula (I) or (II), or a salt thereof, is selected from inflammatory or hyperproliferative skin disorder, immune, autoimmune, inflammatory or infectious disorder, disorder involving IL-8 mediated angiogenesis, metabolic disorder, and neurodegenerative disorder. IL-8 mediated angiogenesis may also be related to complex signal pathways involved in diseases which are believed to have multifactorial causes such as tumors and cancers, e. g., melanoma, thyroid carcinoma, transitional cell carcinoma, trichilemmona, squamous cell carcinoma and breast cancer.

The skin disorder that may be treated or prevented using spirangien according to general formula (I) or (II), or a salt thereof, is preferably selected from palmoplantar pustulosis (PPP), psoriasis, dermatitis, and eczema. These disorders may in particular include plaque psoriasis and guttate type psoriasis, contact dermatitis, eczema erythematosus, and atopic dermatitis.

The immune, autoimmune or inflammatory disorder that can be treated or prevented using spirangien according to general formula (I) or (II), or a salt thereof, is preferably selected from the group consisting of rheumatoid arthritis (RA), psoriatic arthritis, systemic scleroderma and sclerosis, inflammatory bowel disease (IBD), Crohn's disease, ulcerative colitis, acute lung injury, meningitis, encephalitis, uveitis, multiple myeloma, glomerulonephritis, nephritis, asthma, atherosclerosis, leukocyte adhesion deficiency, multiple sclerosis, Raynaud's syndrome, Sjögren's syndrome, juvenile onset diabetes, Reiter's disease, Behcet's disease, immune complex nephritis, IgA nephropathy, IgM polyneuropathies, immune-mediated thrombocytopenias, hemolytic anemia, myasthenia gravis, lupus nephritis, lupus erythematosus, ankylosing spondylitis, pemphigus, Graves' disease, Hashimoto's thyroiditis, small vessel vasculitides, Omen's syndrome, chronic renal failure, autoimmune thyroid disease, acute infectious mononucleosis, HIV, herpes virus associated diseases, human virus infections, coronavirus, other enterovirus, herpes virus, influenza virus, parainfluenza virus, respiratory syncytial virus or adenovirus infection, bacteria pneumonia, wounds, sepsis, cerebral stroke/cerebral edema, ischaemia-reperfusion injury, and hepatitis C.

Other disorders that can be treated or prevented using spirangien according to general formula (I) or (II), or a salt thereof, preferably include a disorder selected from the group consisting of isolated cerebral angiitis, mononeuritis multiplex, acute myocardial infarction, myocarditis, pericarditis, Liebman-Sachs endocarditis, chronic obstructive pulmonary disease (COPD), alveolitis, obliterating bronchiolitis, cystic fibrosis, allergic aspergillosis, Lofflers syndrome, sclerosing cholangiolitis, chronic cyctitis, tubulo-interstial nephritis, severe acute respiratory syndrome (SARS), large vessel vasculitides, giant cell arteritis, polymyalgia rheumatica, Takayasu arteritis, medium-sized vessel vasculitides, polyarteritis nodosa, localized polyarteritis nodosa, Kawasaki disease, small vessel vasculitides, Churg-Strauss syndrome, microscopic polyarteritis, cryoglobulinemic vasculitis, leucocytoclastic angiitis, secondary vasculitides, rheumatoid vasculitis, vasculitis associated with systemic lupus erythematosus or Sjögren's syndrome, isolated sacroileitis, the SAPHO syndrome, disciitis, postoperative disciitis, subacute thyroiditis, cicatricial pemphigoid, dermatitis herpetiformis, subcorneal pustular dermatosis, epidermolysis bullosa acquisita, rosacea, acute febrile dermatosis, granuloma annulare, Sweet's syndrome, pyoderma gangraenosum, acne, acne conglobata, sarcoidosis, relapsing polychondritis, familial Mediterranean fever, panniculitis, erythema nodosum, Weber-Christian's disease, retroperitoneal fibrosis, osteoporosis, osteolytic metastases, gastric cancer, colorectal cancer, urine bladder cancer, diabetes, depression, and Alzheimer's disease.

Preferred, the IL-6 mediated disorder that can be treated or prevented using spirangien according to general formula (I) or (II), or a salt thereof, is diabetes, atherosclerosis, depression, Alzheimer's disease, systemic lupus erythematosus, or rheumatoid arthritis.

Further preferred, the IL-8 mediated disorder that can be treated or prevented using spirangien according to general formula (I) or (II), or a salt thereof, is rheumatoid arthritis, chronic obstructive pulmonary disease, Crohn's disease, psoriasis or palmoplantar pustulosis.

According to the invention, it is also possible to use a combination of spirangiens according to general formula (I) and/or (II), or salts thereof.

The invention is also directed to the use of at least one spirangien according to general formula (I) or (II), or a salt thereof, for the preparation of a medicament intended for the treatment or prevention of an IL-6 or IL-8 mediated disorder. As detailed above, spirangiens according to general formula (I) or (II), or salts thereof, have been widely described in the literature and any one of those or a combination thereof can be employed.

The term "at least one" as used herein is intended to mean one, 2, or more of the respective item or subject.

Moreover, the invention is also directed to a pharmaceutical composition for use in the treatment or prevention of an IL-6 or IL-8 mediated disorder. The pharmaceutical composition of the invention may comprise at least one spirangien according to general formula (I) or (II), or a salt thereof, and, optionally, at least one pharmaceutically acceptable carrier and/or at least one customary excipient.

Carrier substances are, for example, cyclodextrins such as hydroxypropyl β-cyclodextrin, micelles or liposomes, excipients and/or adjuvants. Pharmaceutical compositions may additionally comprise, for example, one or more of water, buffers such as, e.g., neutral buffered saline or phosphate buffered saline, ethanol, mineral oil, vegetable oil, dimethylsulfoxide, carbohydrates such as e.g., glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical compositions provided herein. For instance, the compounds of the invention may advantageously be employed in combination with an antibiotic, anti-fungal, or anti-viral agent, an anti-histamine, a non-steroidal anti-inflammatory drug, a disease modifying anti-rheumatic drug, a cytostatic drug, a drug with smooth muscle activity modulatory activity or mixtures of the aforementioned.

Pharmaceutical compositions may be formulated for any appropriate route of administration, including, for example, topical such as, e.g., transdermal or ocular, oral, buccal, nasal, vaginal, rectal or parenteral administration. The term parenteral as used herein includes subcutaneous, intradermal, intravascular such as, e.g., intravenous, intramuscular, spinal, intracranial, intrathecal, intraocular, periocular, intraorbital, intrasynovial and intraperitoneal injection, as well as any similar injection or infusion technique. In certain embodiments, compositions in a form suitable for oral use are preferred. Such forms include, for example, tablets, troches, lozenges, aqueous or oily suspensions, dispersible powders or granules, emulsion, hard or soft capsules, or syrups or elixirs. Within yet other embodiments, compositions provided herein may be formulated as a lyophilizate.

Compositions intended for oral use may further comprise one or more components such as sweetening agents, flavoring agents, coloring agents and/or preserving agents in order to provide appealing and palatable preparations. Tablets contain the active ingredient in admixture with customary (physiologically acceptable) excipients that are suitable for the manufacture of tablets.

Customary excipients include, for example, inert diluents such as, e.g., calcium carbonate, sodium carbonate, lactose, calcium phosphate or sodium phosphate, granulating and disintegrating agents such as, e.g., corn starch or alginic acid, binding agents such as, e.g., starch, gelatin or acacia, and lubricating agents such as, e.g., magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

The pharmaceutical composition may also comprise a combination of spirangiens according to general formula (I) and/or (II), or salts thereof. The pharmaceutical composition according to the invention can be used for the treatment or prevention of any one of the above disorders, especially for the treatment or prevention of diabetes, atherosclerosis, depression, Alzheimer's disease, systemic lupus erythematosus, rheumatoid arthritis, chronic obstructive pulmonary disease, Crohn's disease, psoriasis or palmoplantar pustulosis.

For the treatment or prevention (prevention) of an IL-6 and/or IL-8 mediated disorder, the dose of the spirangien(s) according to general formula (I) or (II), or (a) salt(s) thereof, may vary within wide limits and may be adjusted to individual requirements. Active compounds according to the present invention are generally administered in a therapeutically effective amount. Preferred doses range from about 0.1 mg to about 140 mg per kilogram of body weight per day, about 0.5 mg to about 7 g per patient per day. The daily dose may be administered as a single dose or in a plurality of doses. The amount of active ingredient that may be combined with the carrier materials to produce a single dosage form will vary depending upon the host treated and the particular mode of administration. Dosage unit forms will generally contain between from about 1 mg to about 500 mg of an active ingredient.

According to the invention, spirangien(s) according to general formula (I) and/or (II), or (a) salt(s) thereof, can be used in the pharmaceutical preparation in a quantity of from 0.01 mg to 2 g, preferably from 1 mg to 1 g, very preferably from 10 mg to 500 mg.

More particularly, spirangien(s) according to general formula (I) and/or (II), or (a) salt(s) thereof, can in particular be administered in doses of from 0.01 mg/kg to 500 mg/kg, preferably 0.1 mg/kg to 100 mg/kg, especially preferred 1 mg/kg to 50 mg/kg.

It will be understood, however, that the specific dose level for any particular patient will depend upon a variety of factors including the activity of the specific compound employed, the age, body weight, general health, sex, diet, time of administration, route of administration, and rate of excretion, drug combination, i.e. other drugs being used to treat the patient, and the severity of the particular disease undergoing therapy.

The invention is also related to a combination preparation comprising spirangiens according to general formula (I) and/or (II), or salts thereof, at least one further active pharmaceutical ingredient, and, optionally, at least one pharmaceutically acceptable carrier(s) and/or at least one customary excipient and at least one further active pharmaceutical ingredient for use in the treatment or prevention of an IL-6 or IL-8 mediated disorder.

Preferably, the further active pharmaceutical ingredient is selected from an anti-inflammatory agent, an anti-infective agent, an anti-depressive agent, a neuroprotective agent, an anti-cancer agent, and an immunoprotective agent.

Preferably, the spirangien(s) according to general formula (I) or (II) used for the treatment of an IL-6 and/or IL-8 mediated disorder will have certain pharmacological properties. Such properties include, but are not limited to oral bioavailability, such that the preferred oral dosage forms discussed above can provide therapeutically effective levels of the compound *in vivo.*

The spirangien(s) according to general formula (I) or (II), or (a) salt(s) thereof, is/are preferably administered to a patient orally or parenterally, and is/are present within at least one body fluid or tissue of the patient.

Accordingly, the present invention further provides methods for preventing or treating patients suffering from an IL-6 or IL-8 mediated disorder.

As used herein, the term "treatment" encompasses both disease-modifying treatment and symptomatic treatment, either of which may be prophylactic, *i.e.*, before the onset of symptoms, in order to prevent, delay or reduce the severity of symptoms, or therapeutic, i.e., after the onset of symptoms, in order to reduce the severity and/or duration of symptoms.

Preferably, the method of preventing or treating an IL-6 or IL-8 mediated disorder according to the invention comprises administering to a subject in need thereof an effective amount of at least one spirangien according to general formula (I) or (II), or a salt thereof.

The method of preventing or treating an IL-6 or IL-8 mediated disorder according to the invention may, moreover, be characterized in that spirangien(s) according to general formula (I) and/or (II), or (a) salt(s) thereof, is/are intended to be administered by oral route, by aerosol route or by injection.

It is especially preferred to combine preferred embodiments of the present invention in any possible manner.

### Brief Description of the Figures

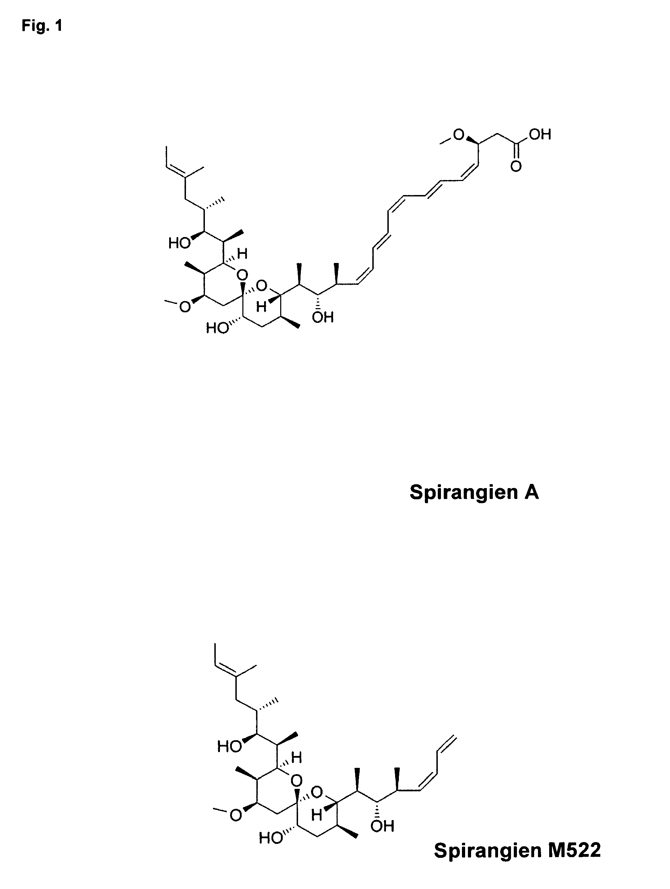

- Figure 1:: Representation of the chemical structures of spirangien A and spirangien M522.
- Figure 2:: A. Graph showing the inhibitory effect of spirangien A and spirangien M522 on the expression of firefly luciferase reporter gene under control of minimal IL-8 promoter. B. Cytotoxicity of various effective concentrations of spirangien A and spirangien M522 on HeLa cells; incubated for 16 h.
- Figure 3:: Inhibitory effect of spirangien A and spirangien M522 on the expression of IL-8. A. IL-8 protein level. B. IL-8 mRNA level.
- Figure 4:: Inhibitory effect of spirangien A and spirangien M522 on the expression of IL-8 and IL6.

The present invention is now further illustrated by the following examples, which are not construed to limit the broad aspects of the invention disclosed herein.

### Examples

### Example 1: Compound Screening

A cell based reporter assay to screen for small molecule inhibitors of IL-8 expression has been established. The assay is based on HeLa S3 cells that are stably transfected with firefly luciferase gene under control of minimal IL-8 promoter. Upon interleukin 1 (IL-1) stimulation, firefly luciferase expression in cells can be detected by luminescence measurement. The assay was performed according to standard protocols and materials were purchased from commercial suppliers and used according to respective manuals supplied therewith. After IL-1 stimulation, compounds spirangien A and spirangien M522 were each added to the cells. After 16 h incubation, cells were lysed and luminescence of the cell lysate was measured.

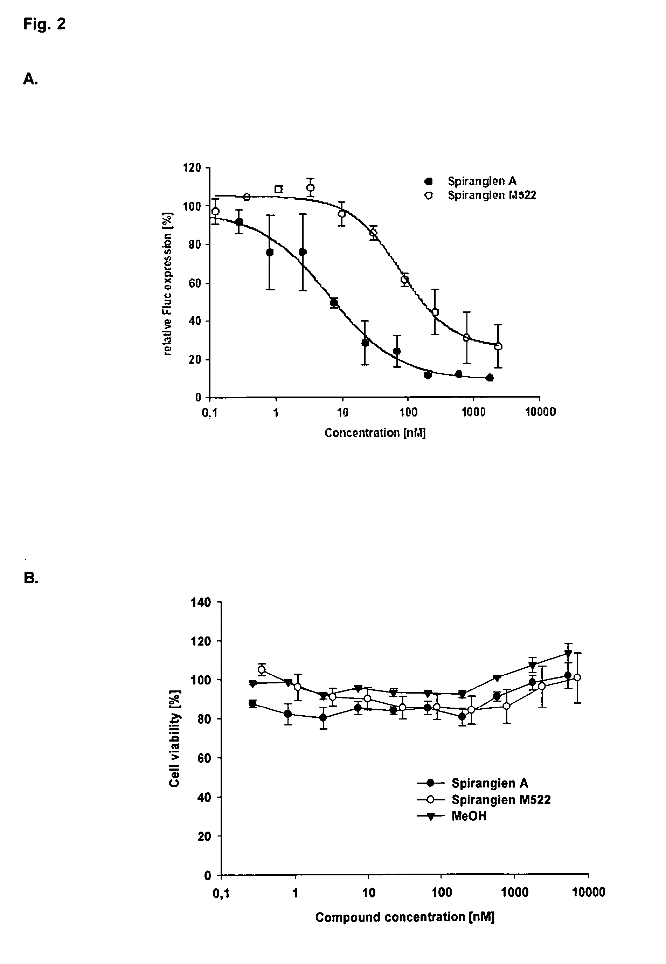

The results are presented in Fig. 2A. These results show that spirangien A and spirangien M522 reduce IL-8 reporter gene expression in a dose dependent manner. Both spirangien A and spirangien M522 have effective IC₅₀ values in nanomolar range:
Spirangien A: IC₅₀ = 6.3 ± 1.7
Spirangien M522 IC₅₀ = 79.6 ± 17.9

Thus, the results indicate that spirangien A and spirangien M522 effectively inhibit IL-8 expression.

### Example 2: Cytotoxicity Test

Cytotoxicity of various effective concentrations of spirangien A and spirangien M522 was determined by MTT assay after 16 h incubation of HeLa cells with the respective test compound at respective concentration. The assay was performed according to standard protocols and materials were purchased from commercial suppliers and used according to respective manuals supplied therewith.

The results are presented in Fig. 2B. These results show that spirangien A and spirangien M522 exhibit no acute toxicity in the effective concentration range.

### Example 3: Expression analysis of IL-8

The effect of spirangien A and spirangien M522 on the expression of IL-8 at the protein and mRNA level was investigated using an ELISA assay and real-time PCR, respectively. The assay was performed with HeLa cells according to standard protocols and materials were purchased from commercial suppliers and used according to respective manuals supplied therewith.

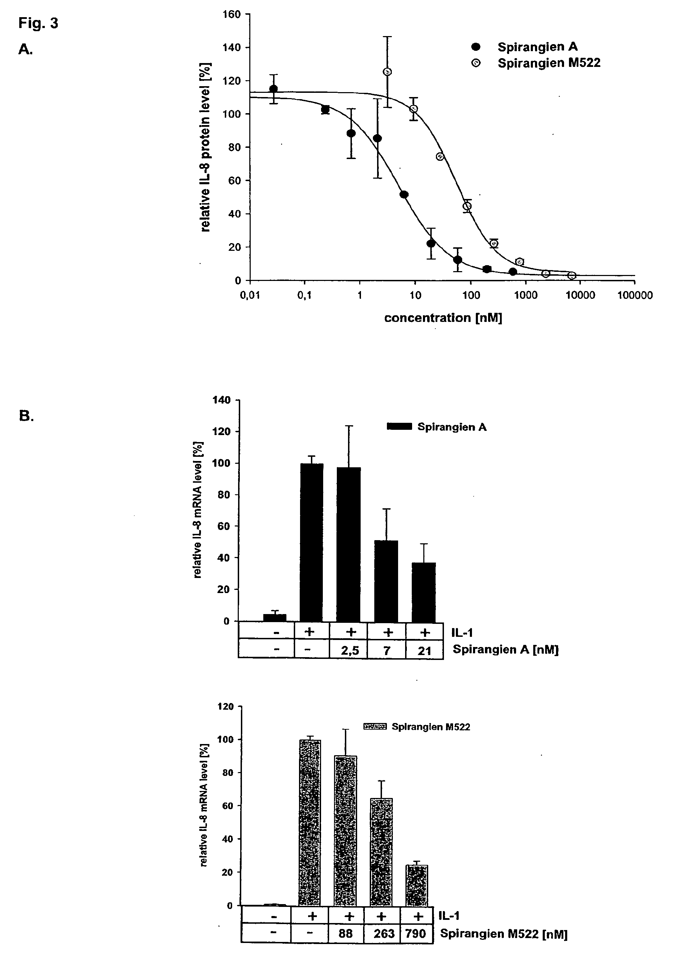

The results are shown in Fig. 3A and 3B. Fig. 3A shows that spirangien A and spirangien M522 reduce the protein expression of IL-8 in a dose dependent manner. As can be taken from Fig. 3B, spirangien A and spirangien M522 reduce the endogenous level of IL-8 mRNA. This shows that spirangien A and spirangien M522 are effective inhibitors of IL-8 expression.

### Example 4: Expression analysis of IL-8 and IL-6

A commercial multiplex cytokine assay was used to determine the effect of spirangien A and spirangien M522 on the expression of other cytokines than IL-8. The assay was performed with HeLa cells according to standard protocols and materials were purchased from commercial suppliers and used according to respective manuals supplied therewith.

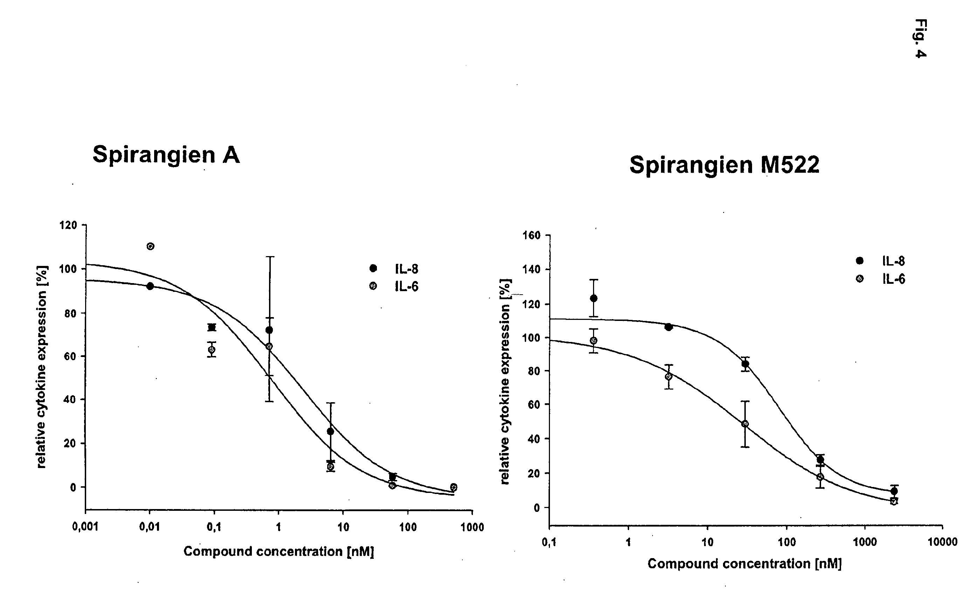

The results are shown in Fig. 4. As can be taken from Fig. 4, spirangien A and spirangien M522 effectively inhibit the expression of IL-6 and IL-8.

The features of the present invention disclosed in the specification, the claims and/or the drawings may both separately and in any combination thereof be material for realizing the invention in various forms thereof.

## Claims

1. Spirangien for use in the treatment or prevention of an IL-6 or IL-8 mediated disorder, wherein the spirangien is a compound according to general formula (I) or (II), or a salt thereof: 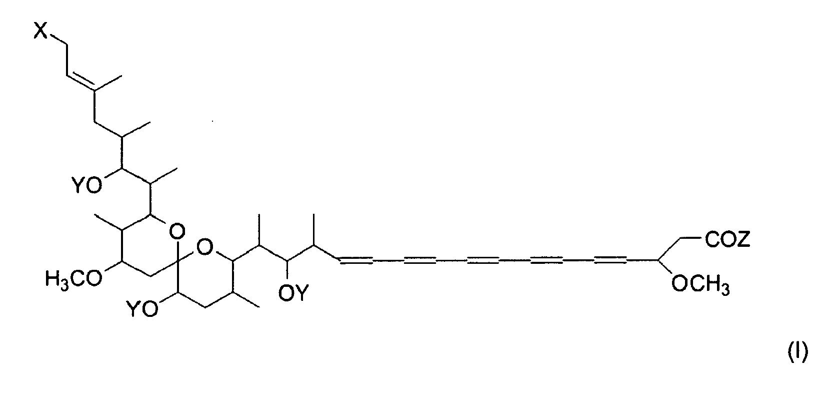 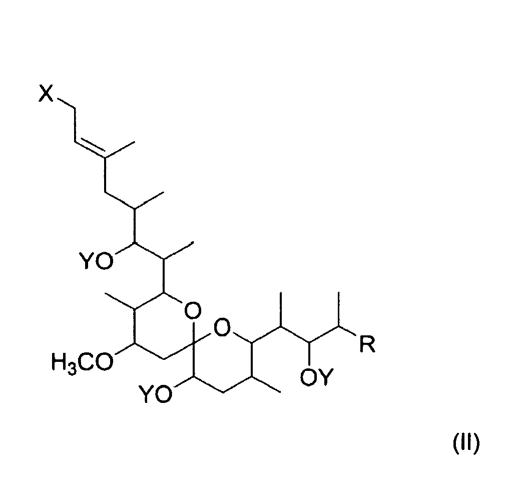 wherein
R is a C₂, C₄ or C₆ alkenyl group;
X is a hydrogen atom or a methyl group;
Y is a hydrogen atom, a C₁-C₅ alkyl, or a C₁-C₅ acyl group;
Z is a OH group; a C₁-C₅ alkoxy group; or a NH₂ group, optionally substituted with one or two C₁-C₅ alkyl groups.

2. The spirangien of Claim 1 for use as defined in Claim 1, wherein the IL-6 or IL-8 mediated disorder is selected from inflammatory or hyperproliferative skin disorder, immune, autoimmune, inflammatory or infectious disorder, disorder involving IL-8 mediated angiogenesis, metabolic disorder, and neurodegenerative disorder.

3. The spirangien of Claim 1 for use as defined in Claim 1 or Claim 2, wherein the skin disorder is selected from palmoplantar pustulosis (PPP), psoriasis, dermatitis, and eczema.

4. The spirangien of Claim 1 for use as defined in Claim 1 or Claim 2, wherein the immune, autoimmune or inflammatory disease is selected from the group consisting of rheumatoid arthritis (RA), psoriatic arthritis, systemic scleroderma and sclerosis, inflammatory bowel disease (IBD), Crohn's disease, ulcerative colitis, acute lung injury, meningitis, encephalitis, uveitis, multiple myeloma, glomerulonephritis, nephritis, asthma, atherosclerosis, leukocyte adhesion deficiency, multiple sclerosis, Raynaud's syndrome, Sjögren's syndrome, juvenile onset diabetes, Reiter's disease, Behcet's disease, immune complex nephritis, IgA nephropathy, IgM polyneuropathies, immune-mediated thrombocytopenias, hemolytic anemia, myasthenia gravis, lupus nephritis, lupus erythematosus, ankylosing spondylitis, pemphigus, Graves' disease, Hashimoto's thyroiditis, small vessel vasculitides, Omen's syndrome, chronic renal failure, autoimmune thyroid disease, acute infectious mononucleosis, HIV, herpes virus associated diseases, human virus infections, coronavirus, other enterovirus, herpes virus, influenza virus, parainfluenza virus, respiratory syncytial virus or adenovirus infection, bacteria pneumonia, wounds, sepsis, cerebral stroke/cerebral edema, ischaemia-reperfusion injury, and hepatitis C.

5. The spirangien of Claim 1 for use as defined in Claim 1 or Claim 2, wherein the disorder is selected from the group consisting of isolated cerebral angiitis, mononeuritis multiplex, acute myocardial infarction, myocarditis, pericarditis, Liebman-Sachs endocarditis, chronic obstructive pulmonary disease (COPD), alveolitis, obliterating bronchiolitis, cystic fibrosis, allergic aspergillosis, Lofflers syndrome, sclerosing cholangiolitis, chronic cyctitis, tubulo-interstial nephritis, severe acute respiratory syndrome (SARS), large vessel vasculitides, giant cell arteritis, polymyalgia rheumatica, Takayasu arteritis, medium-sized vessel vasculitides, polyarteritis nodosa, localized polyarteritis nodosa, Kawasaki disease, small vessel vasculitides, Churg-Strauss syndrome, microscopic polyarteritis, cryoglobulinemic vasculitis, leucocytoclastic angiitis, secondary vasculitides, rheumatoid vasculitis, vasculitis associated with systemic lupus erythematosus or Sjögren's syndrome, isolated sacroileitis, the SAPHO syndrome, disciitis, postoperative disciitis, subacute thyroiditis, cicatricial pemphigoid, dermatitis herpetiformis, subcorneal pustular dermatosis, epidermolysis bullosa acquisita, rosacea, acute febrile dermatosis, granuloma annulare, Sweet's syndrome, pyoderma gangraenosum, acne, acne conglobata, sarcoidosis, relapsing polychondritis, familial Mediterranean fever, panniculitis, erythema nodosum, Weber-Christian's disease, retroperitoneal fibrosis, osteoporosis, osteolytic metastases, gastric cancer, colorectal cancer, urine bladder cancer, diabetes, depression, and Alzheimer's disease.

6. The spirangien of Claim 1 for use as defined in any one of Claims 1 to 5, wherein the IL-6 mediated disorder is diabetes, atherosclerosis, depression, Alzheimer's disease, systemic lupus erythematosus, or rheumatoid arthritis.

7. The spirangien of Claim 1 for use as defined in any one of Claims 1 to 5, wherein the IL-8 mediated disorder is rheumatoid arthritis, chronic obstructive pulmonary disease, Crohn's disease, psoriasis or palmoplantar pustulosis.

8. The spirangien of Claim 1 for use as defined in any one of Claims 1 to 7, wherein the spirangien is a spirangien according to general formula (I), wherein X, Y and Z each represent hydrogen atom (spirangien A); or a spirangien according to general formula (II), wherein R is butadienyl; and X, Y and Z each represent hydrogen atom (spirangien M522).

9. A pharmaceutical composition for use in the treatment or prevention of an IL-6 or IL-8 mediated disorder, which pharmaceutical composition comprises at least one spirangien according to general formula (I) or (II), or a salt thereof: 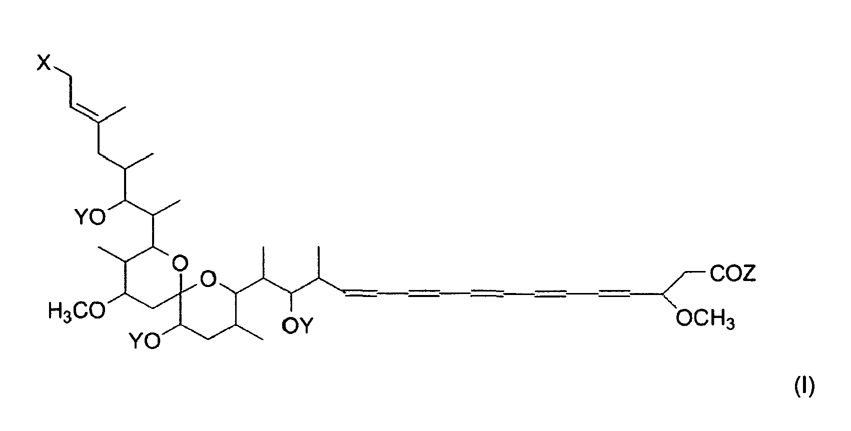 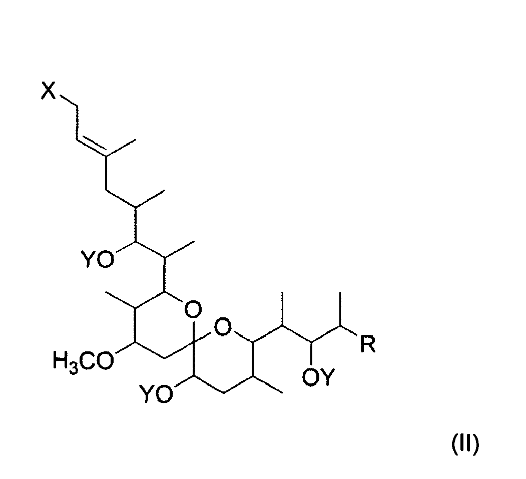 wherein R; X; Y; and Z are as defined in Claim 1; and, optionally, at least one pharmaceutically acceptable carrier and/or at least one customary excipient.

10. The pharmaceutical composition of Claim 8 for use as defined in any one of Claims 2 to 7.

11. A combination preparation for use in the treatment or prevention of an IL-6 or IL-8 mediated disorder, which comprises at least one spirangien according to general formula (I) or (II), or a salt thereof, as defined in Claim 1, at least one further active pharmaceutical ingredient, and, optionally, at least one pharmaceutically acceptable carrier and/or at least one customary excipient.

12. The combination preparation of Claim 11, wherein the further active pharmaceutical ingredient is selected from an anti-inflammatory agent, an anti-infective agent, an anti-depressive agent, a neuroprotective agent, an anti-cancer agent, and an immunoprotective agent.

13. A method of preventing or treating an IL-6 or IL-8 mediated disorder, said method comprising administering to a subject in need thereof an effective amount of at least one spirangien according to general formula (I) or (II), or a salt thereof, as defined in Claim 1.

14. The method of Claim 13, **characterized in that** the spirangien is intended to be administered by oral route, by aerosol route or by injection.
